# EUROPÄISCHE PATENTSCHRIFT

(11) **EP 3 389 496 B1**
(45) Veröffentlichungstag und Bekanntmachung des Hinweises auf die Patenterteilung: **29.01.2020**
(21) Anmeldenummer: 16826294.7
(22) Anmeldetag: 14.12.2016
(51) Int. Cl.: A61B 6/00, A61B 5/00, G06T 11/00

(54) **VERFAHREN ZUR KALIBRIERUNG EINER RÖNTGENAUFNAHME**
METHOD FOR CALIBRATING AN X-RAY IMAGE
PROCÉDÉ POUR CALIBRER UN CLICHÉ RADIOGRAPHIQUE

(30) Priorität: 14.12.2015 DE 102015225130
(43) Veröffentlichungstag der Anmeldung: 24.10.2018
(73) Patentinhaber: Sirona Dental Systems GmbH, 64625 Bensheim (DE)
(72) Erfinder: ABKAI, Ciamak, 68542 Heddesheim (DE)
(74) Vertreter: Özer, Alpdeniz
(86) Internationale Anmeldenummer: PCT/EP2016/080882
(87) Internationale Veröffentlichungsnummer: WO 2017/102782

(56) Entgegenhaltungen:
- DE-A1- 19 620 371
- DE-A1-102009 008 700
- DE-A1-102011 003 984
- US-A1- 2011 255 765

## Beschreibung

### Technisches Gebiet

Die Erfindung betrifft ein Verfahren zur Kalibrierung eines Röntgengeräts zur Vermessung von 2D-Röntgenaufnahmen eines aufzunehmenden Objekts, wobei jede 2D-Röntgenaufnahme aufgenommen wird, indem Röntgenstrahlen mittels einer Röntgenquelle erzeugt werden, das Objekt durchstrahlen und mittels eines Röntgendetektors aufgenommen werden, wobei eine Abbildung des Objekts in der 2D-Röntgenaufnahme durch eine tatsächliche Aufnahmelagebeziehung der Röntgenquelle und des Röntgendetektors relativ zum Objekt bestimmt wird.

### Stand der Technik

Aus dem Stand der Technik sind mehrere Verfahren zur Kalibrierung eines Röntgengerätes zur dreidimensionalen Vermessung, wie ein CT-Röntgengerät oder ein DVT-Röntgengerät, bekannt.

DE 102008035412 A1 offenbart ein Verfahren zur Erstellung einer dentalen 3D-Röntgenaufnahme zumindest eines Teilbereichs eines Objekts, wobei aus mehreren Projektionsaufnahmen während eines Umlaufs um das Objekt das Volumen als 3D-Röntgenbild erstellt wird. Dabei wird vor der Erstellung der Röntgenaufnahme mindestens ein Teil des Objekts in einer bildlichen Darstellung angezeigt, wobei die relative Lage der bildlichen Darstellung zu der aktuellen Geräte- und Patientenposition bekannt ist. Das von der Positionierung des Objekts bezüglich des Gerätes und von der Wahl der Einstellungs- und/oder Steuerungsdaten abhängige aufzunehmende Volumen wird in die bildliche Darstellung zumindest näherungsweise lagerichtig eingeblendet und bei der Veränderung der Lage und/oder Größe des aufzunehmenden Volumens in der bildlichen Darstellung werden die Einstellungs -und/oder Steuerungsdaten zur Erstellung der 3D-Röntgenaufnahme bestimmt. Das aufzunehmende Volumen wird in der bildlichen Darstellung lediglich schematisch als ein ungefährer Bereich eingeblendet. Die Aufnahmewinkel beider Aufnahmen können unterschiedlich sein. Die bildliche Darstellung kann auch eine vorhandene 3D-Aufnahme, wie eine 3D-Röntgenaufnahme sein. Für die lagerichtige Einblendung des aufzunehmenden Volumens in die bildliche Darstellung wird eine Lageregistrierung durchgeführt, wobei die relative Position des Gerätes zum Patienten bei der bildlichen Darstellung mit der aktuellen Geräte -und Patientenposition verglichen wird, um die Position des aufzunehmenden Volumens in der bildlichen Darstellung korrekt darzustellen.

DE 102009008700 A1 offenbart ein Verfahren zur Erstellung von Röntgenbildern mittels eines Röntgengeräts, aufweisend einen verstellbaren C-Bogen, welcher einen Röntgendetektor und eine Röntgenquelle bewegt. Die Verformung des C-Bogens wird mittels Dehnmeßstreifen ermittelt und bei der 3D-Rekonstruktion berücksichtigt.

DE 102011003984 A1 offenbart ein Verfahren zur Lagebestimmung eines an einem Körper eines Patienten eingebrachten Fremdkörpers, wobei von dem Fremdkörper ein virtuelles 3D-Modell vorhanden ist, wobei das virtuelle 3D-Modell mit einem 2D-Röntgenbild verglichen wird, wobei die Lage des Fremdkörpers im 2D-Röntgenbild ermittelt wird, wobei anhand der Lage des Abbildes und des vorhandenen 3D-Modells die Lage des Körpers im Raum bzw. relativ zu einem Organ bestimmt wird.

DE 19620371 A1 offenbart ein Röntgenaufnahmeverfahren, bei dem mit einer ersten bildgebenden Einrichtung eine Serie von zweidimensionalen Röntgenaufnahmen angefertigt wird, bei denen das Objekt aus unterschiedlichen Perspektiven projiziert wird. Aus den einzelnen zweidimensionalen Röntgenaufnahmen wird ein Überlagerungsbild erzeugt, um anatomische Strukturen in geometrisch korrekter Zuordnung als dreidimensionale Objekte wiederzugeben.

US 2011/0255765 A1 offenbart ein System und ein Verfahren zum Entfernen von Artefakten aus Röntgenbildern der Zähne eines Patienten, wobei das System einen Oberflächenscanner aufweist, der eine Oberflächenaufnahme der Zähne des Patienten erzeugt. Die Oberflächendaten der Oberflächenaufnahme und die Volumendaten der CT-Aufnahme werden angepasst, zueinander ausgerichtet und überlagert, um einen kombinierten Datensatz zu erzeugen. Datenpunkte der Volumendaten, die über die Oberfläche der Zähne in den Oberflächendaten hinausgehen, werden identifiziert und entfernt, so dass dadurch durch Metallteile erzeugte Artefakte entfernt werden.

Bei einer Ausführungsform wird das Oberflächenmodell der Oberflächendaten projiziert, um Vorwärtsprojektionsdaten in dem gleichen zweidimensionalen Format wie die CT-Projektionsdaten zu erzeugen. Die Vorwärtsprojektionsdaten werden mit den CT-Projektionsdaten kombiniert, um die Artefakte zu identifizieren. Eine manuelle Vorpositionierung der beiden Projektionsdaten zueinander ist nicht erforderlich.

Bei einem DVT-Verfahren werden die Röntgenröhre und ein gegenüberliegender digitaler Bildsensor um das Objekt rotiert, wobei während eines teilweisen Umlaufs der Röntgenröhre mehrere zweidimensionale Röntgenaufnahmen aus unterschiedlichen Aufnahmewinkeln erzeugt werden. Anschließend wird aus den einzelnen zweidimensionalen Röntgenaufnahmen durch ein Rekonstruktionsverfahren ein dreidimensionales Röntgenbild rekonstruiert. Beim Rekonstruktionsverfahren ist eine korrekte Aufnahmegeometrie beziehungsweise Aufnahmelagebeziehung der Röntgenröhre und des Röntgendetektors relativ zum Objekt wesentlich. Zur Bestimmung dieser Aufnahmelagebeziehung werden typischerweise Kalibrationsphantome verwendet. Solche Kalibrationsmessungen werden ab Werk und in regelmäßigen Abständen nach der Inbetriebnahme des Röntgengerätes durchgeführt.

Ein Nachteil dieses Verfahrens besteht darin, dass die ursprüngliche Lagebeziehung, die bei der Kalibrierung festgestellt wurde, sich beispielsweise durch die Abnutzung der Mechanik des Röntgengerätes oder durch sich ändernde Reibkräfte der Antriebe des Röntgengerätes verändern kann. Durch die Veränderung der Lagebeziehung stimmt also die tatsächliche Lagebeziehung des Röntgenstrahlers und des Röntgendetektors relativ zum Objekt nicht mit der vorgegebenen Lagebeziehung aus der Kalibrierungsmessung überein, sodass dadurch der Rekonstruierungsprozess verfälscht wird und Artefakte, wie Verwischungen von Details, Streifen und/oder Schatten, in der rekonstruierten dreidimensionalen Röntgenaufnahme auftreten können. Bei einer Patientenbewegung während der Aufnahme können auch Bewegungsartefakte entstehen.

Auch bei Geräten zur Erstellung von Panoramaschichtaufnahmen oder für die intraoralen Röntgenaufnahmen, die auf eine bekannten kalibrierte Lagebeziehung angewiesen sind, werden durch fehlerhafte Aufnahmelagebeziehungen weitere Fehler in den Röntgenaufnahmen induziert.

Die Aufgabe der vorliegenden Erfindung besteht daher darin, ein Verfahren zur Kalibrierung eines Röntgengeräts bereitzustellen, das auch ohne Verwendung eines Kalibrierungsphantomes eine fehlerfreie Vermessung des Objekts gewährleistet.

### Darstellung der Erfindung

Die Erfindung betrifft ein Verfahren zur Kalibrierung mindestens einer 2D-Röntgenaufnahme eines aufzunehmenden Objekts, die mittels eines Röntgengeräts aufgenommen wurde, indem Röntgenstrahlen, die mittels einer Röntgenquelle erzeugt werden, das Objekt durchstrahlen und mittels eines Röntgendetektors aufgenommen werden. Dabei wird ein bereits vorhandenes 3D-Modell umfassend eine Struktur des Objekts mit der 2D-Röntgenaufnahme verglichen, wobei eine tatsächliche Aufnahmelagebeziehung der Röntgenquelle und des Röntgendetektors relativ zum Objekt und/oder relativ zueinander für die 2D-Röntgenaufnahme bestimmt wird.

Die 2D-Röntgenaufnahmen können beispielsweise mittels eines DVT-Röntgengerätes aufgenommen werden. Dabei werden also während eines Umlaufes mehrere 2D-Röntgenaufnahmen aus unterschiedlichen Aufnahmewinkeln erzeugt. Die Röntgenquelle wird dabei in der Regel um 180° bis 360° um das Objekt rotiert, wobei die Röntgenquelle einen kegelförmigen Fächer aus Röntgenstrahlen erzeugt, der meist gepulst ist. Die Röntgenstrahlen durchdringen das dreidimensionale Objekt und erzeugen am Röntgendetektor ein abgeschwächtes Grauwerte-Röntgenbild als eine 2D-Röntgenaufnahme für den jeweiligen Aufnahmewinkel bzw. den jeweiligen Aufnahmezeitpunkt.

Der Vergleich zwischen dem vorhandenen 3D-Modell und der 2D-Röntgenaufnahme kann beispielsweise dadurch erfolgen, dass Punkte der Struktur im 3D-Modell den entsprechenden Punkten der Struktur in der 2D-Röntgenaufnahme zugeordnet werden.

Die ermittelte tatsächliche Aufnahmelagebeziehung der Röntgenquelle und des Röntgendetektors relativ zum Objekt oder relativ zueinander kann abgespeichert werden und für die Korrektur, Rekonstruktion, Optimierung und/oder Berechnung späterer Röntgenaufnahmen verwendet werden.

Auf diese Weise muss das vorliegende Verfahren zur Kalibrierung einmalig oder in festgelegten Zeitabständen zur Überprüfung der Kalibrierung durchgeführt werden.

Ein Vorteil dieses Verfahrens besteht darin, dass zur Vermessung eines Objekts keine vorangehende Kalibrierung mittels eines Kalibrierungsphantomes erforderlich ist. Dadurch wird also der Wartungsaufwand gemindert.

Ein weiterer Vorteil dieses Verfahrens besteht darin, dass für jede einzelne 2D-Röntgenaufnahme aus einem bestimmten Aufnahmewinkel eine Kalibrierung durch Vergleich mit dem 3D-Modell durchgeführt wird, sodass auch unvorhergesehene Störungen des Röntgengerätes, die zur Änderung der Aufnahmelagebeziehung führen, unmittelbar korrigiert werden.

Ein weiterer Vorteil dieses Verfahrens besteht darin, dass eine 3D-Röntgenaufnahme eines Patienten erzeugt werden kann, der sich während des Umlaufes bewegt. Beispielsweise kann der gesamte Unterkiefer als die aufnehmende Struktur auch bei einer seitlichen Bewegung des Kopfes oder bei einer Kaubewegung durch den Patienten scharf, ohne Bewegungsartefakte, rekonstruiert werden. Denn durch den Vergleich der einzelnen 2D-Röntgenaufnahmen mit der virtuellen Projektion des 3D-Modells in der virtuellen 2D-Projektionsaufnahme wird die genaue Aufnahmelagebeziehung dieser Struktur, wie beispielsweise eines Unterkiefers, relativ zur Röntgenquelle und zum Röntgendetektor bestimmt. Damit wird also die Kalibrierung in Bezug auf die abgebildete Struktur des Objekts, wie einen Unterkiefer, trotz Patientenbewegung ideal bestimmt.

Beim Vergleich des 3D-Modells mit der 2D-Röntgenaufnahme wird durch die Anwendung eines virtuellen Projektionsverfahrens auf mindestens einen Teil des 3D-Modells unter Berücksichtigung einer vorgegebenen Aufnahmelagebeziehung eine virtuelle 2D-Projektionsaufnahme erzeugt, wobei die Struktur in der 2D-Röntgenaufnahme mit der Struktur in der virtuellen 2D-Projektionsaufnahme verglichen wird.

Beim virtuellen Projektionsverfahren wird also die Aufnahmegeometrie für die jeweilige 2D-Röntgenaufnahme mittels eines Computers simuliert und dabei die entsprechende virtuelle 2D-Projektionsaufnahme erzeugt. Beim Projektionsverfahren wird also die Aufnahmelagebeziehung einer virtuellen Röntgenquelle und eines virtuellen Röntgendetektors relativ zum 3D-Modell der Struktur simuliert, wobei simuliert wird, wie die Röntgenstrahlen in Form eines kegelförmigen Fächers das 3D-Modell virtuell durchstrahlen und auf dem virtuellen Röntgendetektor abbilden. Dies hat also zur Folge, dass die Form einer Abbildung der Struktur in der 2D-Röntgenaufnahme mit einer virtuellen Projektion der Struktur in der virtuellen 2D-Projektionsaufnahme bei gleicher Aufnahmelagebeziehung übereinstimmt.

Bei einem alternativen Verfahren kann der Vergleich zwischen dem 3D-Modell und der 2D-Röntgenaufnahme dadurch erfolgen, dass ein virtuelles Rückprojektionsverfahren auf die 2D-Röntgenaufnahme angewendet wird, wobei die 2D-Röntgenaufnahme also in einen 3D-Raum abgebildet wird. Anschließend wird diese Projektion mit dem vorhandenen 3D-Modell verglichen.

Beim Vergleich der Struktur in der 2D-Röntgenaufnahme mit der virtuellen 2D-Projektionsaufnahme werden Abweichungen festgestellt, wobei die Aufnahmelagebeziehung Schritt für Schritt im Rahmen eines Optimierungsverfahrens geändert wird und nach jeder Änderung eine erneute virtuelle 2D-Projektionsaufnahme erzeugt wird, bis die Abweichungen kleiner als ein festgelegter Schwellenwert sind.

Die vorgegebene Aufnahmelagebeziehung der Röntgenquelle und des Röntgendetektors relativ zum Objekt kann aus einer Kalibrierung oder einer späteren Kalibrierung stammen oder mathematisch anhand eines Modells ermittelt werden. Die vorgegebene Aufnahmelagebeziehung ist also eine Startlösung, ausgehend von der unter Anwendung des Optimierungsverfahrens die tatsächliche Aufnahmelagebeziehung ermittelt wird. Beim Optimierungsverfahren kann ein so genannter Kalman-Filter verwendet werden, wobei Startlösungen stabilisiert werden. Ein Kalman-Filter dient zum Entfernen der von Messgeräten verursachten Störungen.

Der Schwellenwert wird so festgelegt, dass bei Unterschreitung dieses Schwellenwerts die Struktur in der 2D-Röntgenaufnahme mit der virtuellen 2D-Projektionsaufnahme möglichst übereinstimmt, so dass die ermittelte Aufnahmelagebeziehung nach dem Ablauf des Optimierungsverfahrens der tatsächlichen Aufnahmelagebeziehung der jeweiligen 2D-Röntgenaufnahme in Bezug auf die Struktur des abgebildeten Objekts entspricht oder zumindest ähnlich ist.

Der Vergleich kann mittels eines Vergleichsoperators erfolgen. Beim Optimierungsverfahren wird dann der Vergleichsoperator optimiert. Das Optimierungsverfahren kann beispielsweise abgeschlossen werden, nachdem ein vorgegebener Schwellenwert erreicht wurde. Der Schwellenwert der Qualitätsgenauigkeit der Optimierung kann beispielsweise 10 % der Kalibrierungsgenauigkeit des jeweiligen Röntgengeräts entsprechen.

Das Optimierungsverfahren kann entweder den Vergleichsoperator direkt optimieren oder die Abweichungen zwischen den Strukturen in der virtuellen 2D-Projektionsaufnahme und in der 2D-Röntgenaufnahme optimieren.

Vorteilhafterweise können die 2D-Röntgenaufnahmen bei einer Bewegung der Röntgenquelle und des Röntgendetektors um das Objekt schrittweise aus unterschiedlichen Aufnahmewinkeln aufgenommen werden, wobei aus den aufgenommenen 2D-Röntgenaufnahmen durch die Anwendung eines Rekonstruktionsverfahrens in Kenntnis der ermittelten tatsächlichen Aufnahmelagebeziehungen der 2D-Röntgenaufnahmen eine 3D-Röntgenaufnahme des Objekts oder eine Panoramaschichtaufnahme des Objekts erzeugt wird.

Die Bewegung der Röntgenquelle und des Röntgendetektors um das Objekt kann dabei eine kreisförmige Rotation in Form eines teilweisen Umlaufs um das Objekt oder auch eine andere Bewegung durch Variation von Orientierung und Position von Röntgenquelle und Röntgendetektor im Bezug zum Objekt darstellen.

Dadurch kann das vorliegende Verfahren also bei der Kalibrierung eines DVT-Röntgengerätes oder eines CT-Röntgengerätes verwendet werden. Die Kalibrierung der Aufnahmelagebeziehung wird also bei jeder einzelnen 2D-Röntgenaufnahme überprüft und korrigiert. Dies ermöglicht also eine dynamische Überprüfung, sodass auch plötzliche mechanische Störungen des Gerätes berücksichtigt werden. Beim Rekonstruktionsverfahren entspricht das Grauwertebild in der 2D-Röntgenaufnahme der Summe der Absorptionen entlang eines gemessenen Röntgenpfades durch das Objekt. Der Röntgenpfad wird dann in kleine Voxel zerlegt. Bei der Rückprojektion wird jeweils der Messwert entlang jedes gemessenen Röntgenpfades bestmöglich auf die entlang des Pfades liegenden Voxel verteilt. Dies erfolgt für die 2D-Röntgenaufnahmen aus verschiedenen Aufnahmewinkeln, sodass man als Ergebnis eine gute Schätzung der 3D-Röntgenaufnahme des dargestellten Objekts erhält.

Bei der Rekonstruktion einer Panoramaschichtaufnahme wird unter Verwendung eines Computers ausgehend von den 2D-Röntgenaufnahmen aus unterschiedlichen Aufnahmerichtungen eine Panoramaschichtaufnahme berechnet.

Bei der Startlösung handelt es sich also um eine geplante Bahn der Röntgenquelle und des Röntgendetektors um das Objekt, die beispielsweise auch von einer Werkskalkulation übernommen werden kann. Diese Bahn, die die einzelnen Lagepositionen der Röntgenquelle und des Röntgendetektors relativ zur Objekt für die einzelnen Aufnahmewinkel miteinander verbindet, wird dann im Laufe des Optimierungsverfahrens optimiert bzw. schrittweise feiner bestimmt, bis die tatsächliche Bahn ermittelt wird. Die tatsächliche Bahn wird dann bei der Rekonstruktion verwendet, so dass die fehlerfreie 3D-Röntgenaufnahme oder eine Panoramaschichtaufnahme rekonstruiert wird.

Vorteilhafterweise kann die Struktur des Objekts ein Oberkiefer, ein Unterkiefer, eine Gruppe von Zähnen, ein Zahnersatzteil, eine Füllung, ein Inlay, das gesamte Objekt, einen Teil des Objekts, ein Patientenkopf und/oder ein einzelner Zahn sein.

Das Objekt kann also auch ein Teil eines Kiefers bestehend aus mehreren Zähnen und eines Zahnersatzteils sein.

Vorteilhafterweise kann das vorhandene 3D-Modell der Struktur mittels eines optischen dreidimensionalen Oberflächenvermessungsverfahrens aufgenommen sein, wobei das 3D-Modell lediglich eine Oberfläche der Struktur enthält. Beim Vergleich wird dann ein Oberflächenrand der Struktur in der 2D-Röntgenaufnahme mit einem Oberflächenrand der Struktur in der virtuellen 2D-Projektionsaufnahme verglichen.

Das vorhandene 3D-Modell der Struktur kann dabei die gesamte Oberfläche der Struktur oder auch nur einen Teil der Oberfläche der Struktur umfassen. Bei der Vermessung mittels eines dreidimensionalen Oberflächenscanners können beispielsweise nur die sichtbaren Oberflächen der Zähne vermessen werden. Das Oberflächenvermessungsverfahren kann beispielsweise ein Streifenprojektionsverfahren, ein konfokales Messverfahren oder ein Laserscanverfahren sein. Bei der virtuellen Projektion des 3D-Modells entsteht also der Oberflächenrand der Struktur, der mit dem Oberflächenrand der Struktur in der 2D-Röntgenaufnahme verglichen werden kann. Beispielsweise kann der Rand der Zähne oder auch die Vertiefungen der Zähne, die in den 2D-Röntgenaufnahmen deutlich sichtbar sind, für den Vergleich verwendet werden.

Das vorhandene 3D-Modell kann auch lediglich eine Punktwolke darstellen, die die Oberfläche oder markante Punkte der Struktur darstellt.

Vorteilhafterweise kann das vorhandene 3D-Modell der Struktur erzeugt wurde, indem ein Abdruck der Struktur mittels eines optischen dreidimensionalen Oberflächenvermessungsverfahrens aufgenommen wurde, wobei das 3D-Modell lediglich eine Oberfläche der Struktur enthält, wobei beim Vergleich ein Oberflächenrand der Struktur in der 2D-Röntgenaufnahme mit einem Oberflächenrand der Struktur in der virtuellen 2D-Projektionsaufnahme verglichen wird.

Dadurch wird also lediglich ein Abdruck der Struktur vermessen, sodass daraus die Oberfläche dieser Struktur ermittelt wird. Dabei kann also beispielsweise der Abdruck einer oder mehrerer Zähne vermessen werden.

Vorteilhafterweise kann das vorhandene 3D-Modell der Struktur mittels eines dreidimensionalen Volumenvermessungsverfahrens, insbesondere mittels eines MRT-Verfahrens, CT-Verfahrens oder DVT-Verfahrens, aufgenommen sein und Volumendaten der Struktur enthalten, wobei beim Vergleich die Struktur in der 2D-Röntgenaufnahme mit der simulierten Projektion der Struktur in der virtuellen 2D-Projektionsaufnahme verglichen wird.

Das 3D-Modell kann also auch Volumendaten mit Informationen über den inneren Aufbau der Struktur aufweisen. Dabei können also für den Vergleich auch Unterstrukturen innerhalb der Struktur, also beispielsweise die Trennfläche zwischen einem Zahn und dem umgebenden Zahnfleisch oder der Verlauf der Zahnwurzeln oder des Kieferknochens verwendet werden.

Das Magnetresonanztomographieverfahren (MRT) beruht physikalisch auf dem Prinzip der Kernspinresonanz, wobei die Trennfläche zwischen weichem Gewebe und hartem Gewebe, wie zwischen Zähnen und dem umgebenden Zahnfleisch, deutlicher als bei einer 3D-Röntgenaufnahme dargestellt wird.

Das Computertomographieverfahren (CT) beruht auf einer Rekonstruktion einer 3D-Röntgenaufnahme aus einzelnen 2D-Röntgenaufnahmen des Objekts aus unterschiedlichen Aufnahmewinkeln, wobei als Röntgendetektor ein Mehrzeilen-Detektor verwendet wird.

Bei der digitalen Volumentomographie (DVT) wird die 3D-Röntgenaufnahme ebenfalls aus den einzelnen 2D-Röntgenaufnahmen unterschiedlicher Aufnahmewinkel rekonstruiert, wobei als Röntgendetektor ein Flatpanel-Detektor verwendet wird.

Vorteilhafterweise kann beim Projektionsverfahren nicht nur die Aufnahmelagebeziehung der Röntgenquelle und des Röntgendetektors relativ zum Objekt, sondern auch die Dicke der aufzunehmenden Struktur und damit die Röntgenschwächung durch die Struktur und/oder das Material der Struktur und damit die davon abhängige Röntgenschwächung berücksichtigt werden. D.h. der Vergleich einer virtuellen Projektionsaufnahme und einer 2D-Röntgenaufnahme beschränkt sich nicht nur auf den Rand einer Struktur, sondern auf weitere Bildinhalte.

Durch die Berücksichtigung der Dicke und des Materials der Struktur ist also eine verbesserte Simulation der Projektion in der virtuellen 2D-Projektionsaufnahme möglich. Bei der Vermessung der Struktur mittels eines optischen Oberflächenscanners kann beispielsweise bestimmt werden, ob es sich bei der Struktur um einen natürlichen Zahn, ein Zahnersatzteil aus Keramik, aus Gold oder aus Kunststoff handelt. Dabei wird also der jeweilige Faktor für die Röntgenschwächung für Gold, Kunststoff oder Keramik berücksichtigt.

Vorteilhafterweise kann bei der Durchführung des Optimierungsverfahrens als Startlösung die vorgegebene Aufnahmelagebeziehung aus einer bekannten Werkskalibrierung verwendet werden.

Dadurch wird also eine Startlösung verwendet, die bereits sehr nahe an der tatsächlichen Aufnahmelagebeziehung liegt.

Dadurch werden also die Dauer und der Rechenaufwand für das Optimierungsverfahren vermindert.

Vorteilhafterweise kann beim Vergleich der 2D-Röntgenaufnahme mit der virtuellen 2D-Projektionsaufnahme zur Bestimmung der Abweichungen ein Ähnlichkeitsmaß berechnet werden, wobei ein Gradienten-Differenzen-Verfahren, ein direktes Differenzen-Verfahren, ein Korrelationen-Verfahren, ein Kreuzkorrelationen-Verfahren erster bzw. höherer Ordnung, ein statistisches Verfahren oder ein Verfahren der geringsten quadratischen Fehler verwendet wird.

Bei der Anwendung der genannten Verfahren werden also übereinstimmende Muster in der 2D-Röntgenaufnahme und in der virtuellen 2D-Projektionsaufnahme, wie beispielsweise der Rand der Zähne, miteinander verglichen, um dadurch die tat-, sächliche Aufnahmelagebeziehung zu bestimmen. Das Ähnlichkeitsmaß steigt dabei bei zunehmender Ähnlichkeit dieser Muster. Bei einer übereinstimmenden Aufnahmelagebeziehung in der 2D-Röntgenaufnahme und in der simulierten 2D-Projektionsaufnahme sollten die Muster übereinstimmen oder sich zumindest teilweise ähneln. In diesem Fall ist also das Ähnlichkeitsmaß maximal, sodass das Optimierungsverfahren beendet werden kann.

Das statistische Verfahren kann beispielsweise ein sogenanntes Mutual-Information-Verfahren sein.

Vorteilhafterweise kann durch die Anwendung des Optimierungsverfahrens das Ähnlichkeitsmaß größer und damit die Abweichungen geringer werden, bis ein Optimum und damit die tatsächliche Aufnahmebeziehung ermittelt werden.

Dadurch nähern sich die Lösungen für die Aufnahmebeziehung bei der Anwendung des Optimierungsverfahrens der optimalen Lösung an.

Vorteilhafterweise kann die Änderung der Aufnahmelagebeziehung im Verlauf des Optimierungsverfahrens durch eine Transformationsmatrix beschrieben werden.

Durch die Anwendung der Transformationsmatrix kann also das 3D-Modell schrittweise virtuell relativ zur Röntgenquelle und zum Röntgendetektor verschoben oder rotiert werden.

Vorteilhafterweise kann in einem ersten Schritt eine erste Struktur des Objekts gewählt werden, um nach dem vorliegenden Verfahren die ersten tatsächlichen Aufnahmelagebeziehungen der 2D-Röntgenaufnahmen für diese erste Struktur zu bestimmen, wobei in einem zweiten Schritt eine zweite Struktur des Objekts gewählt wird, um nach dem vorliegenden Verfahren die zweiten tatsächlichen Aufnahmelagebeziehungen der 2D-Röntgenaufnahmen für diese zweite Struktur zu bestimmen.

Dadurch werden also für die jeweils gewählte Struktur die entsprechenden tatsächlichen Aufnahmelagebeziehungen bestimmt.

Vorteilhafterweise kann unter Verwendung der ersten tatsächlichen Aufnahmelagebeziehungen eine erste 3D-Röntgenaufnahme rekonstruiert werden und unter Verwendung der zweiten tatsächlichen Aufnahmelagebeziehungen eine zweite 3D-Röntgenaufnahme rekonstruiert wird, wobei anschließend ein erster Bereich in der ersten 3D-Röntgenaufnahme, der die erste Struktur scharf abbildet, mit einem zweiten Bereich in der zweiten 3D-Röntgenaufnahme, der die zweite Struktur scharf abbildet, zu einer gesamten 3D-Röntgenaufnahme des Objekts zusammengefügt werden.

Dadurch wird also zumindest die gewählte Struktur in der entsprechenden 3D-Röntgenaufnahme dieser Struktur scharf abgebildet.

Vorteilhafterweise kann die erste Struktur ein Unterkiefer oder ein Teil des Unterkiefers und die zweite Struktur ein Oberkiefer oder ein Teil des Oberkiefers sein.

Dadurch wird also die erste 3D-Röntgenaufnahme mit dem Unterkiefer als gewählte Struktur erzeugt, sodass auch bei einer Bewegung des Unterkiefers während der Aufnahme der Unterkiefer scharf abgebildet wird, wobei der Oberkiefer und der restliche Patientenkopf unscharf abgebildet werden. In der zweiten 3D-Röntgenaufnahme werden demnach der Oberkiefer und der restliche Patientenkopf scharf abgebildet, wobei der Unterkiefer unscharf abgebildet wird. Die scharfen Bereiche beider 3D-Röntgenaufnahmen können dann zu einer gesamten 3D-Röntgenaufnahme zusammengefügt werden, die sowohl den Unterkiefer als auch den Oberkiefer scharf abbildet.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens, die
- Fig. 2: eine Skizze zur Verdeutlichung des Optimierungsverfahrens bei der Startlösung, die
- Fig. 3: eine Skizze zur Verdeutlichung des Optimierungsverfahrens bei der finalen Lösung. Ausführungsbeispiele

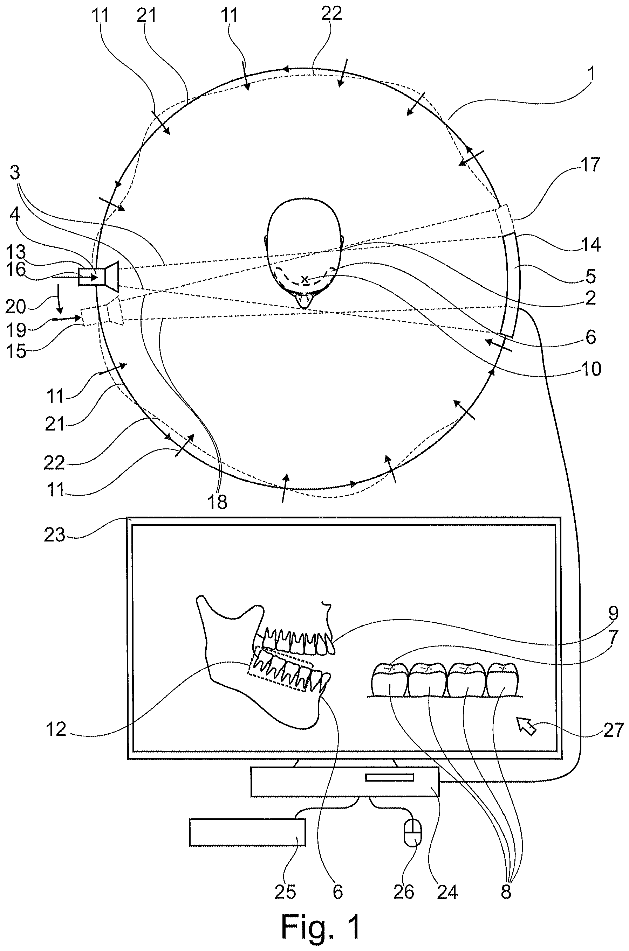

Die Fig. 1 zeigt eine Skizze zur Verdeutlichung des vorliegenden Verfahrens zur Kalibrierung eines Röntgengerätes 1 zur Vermessung von 2D-Röntgenaufnahmen eines aufzunehmenden Objektes 2, wie eines Patientenkopfes. Jede 2D-Röntgenaufnahme wird aufgenommen, indem Röntgenstrahlen 3 in Form eines kegelförmigen Fächers mittels einer Röntgenquelle 4 erzeugt werden, das Objekt 2 durchstrahlen und mittels eines Röntgendetektors 5, wie eines Flatpanel-Detektors, aufgenommen werden. Das Objekt 2 enthält dabei eine aufzunehmende Struktur 6, wie einen Unterkiefer, der gestrichelt dargestellt ist. Zur aufzunehmenden Struktur 6, also dem Unterkiefer, ist bereits ein 3D-Modell 7 vorhanden, das mittels eines optischen dreidimensionalen Oberflächenvermessungsverfahrens aufgenommen wurde. Im vorliegenden Fall umfasst das vorhandene 3D-Modell 7 lediglich vier Backenzähne 8 auf der rechten Seite des Unterkiefers 6. Das 3D-Modell kann beispielsweise mittels einer handgehaltenen 3D-Dentalkamera aufgenommen sein, die auf einem Streifenprojektionsverfahren oder einem konfokalen Vermessungsverfahren beruht. Das 3D-Modell kann auch erzeugt werden, indem zunächst ein Abdruck der Zähne 8 abgenommen wird und anschließend dieser Abdruck mittels der handgehaltenen Dentalkamera vermessen wird. Im 3D-Modell 7 sind also lediglich sichtbare Oberflächen der Backenzähne 8 enthalten. Bei der Vermessung einer 3D-Röntgenaufnahme 9 werden die Röntgenquelle 4 und der Röntgendetektor 5 schrittweise um einen Rotationspunkt 10 innerhalb eines Messvolumens rotiert, wobei aus unterschiedlichen Aufnahmewinkeln 11, die durch Pfeile dargestellt sind, einzelne 2D-Röntgenaufnahmen des Objekts 2 und damit der Struktur 6 aufgenommen werden, wobei aus den aufgenommenen 2D-Röntgenaufnahmen aus den unterschiedlichen Aufnahmewinkeln 11 durch Anwendung eines Rekonstruktionsverfahrens die 3D-Röntgenaufnahme 9 des Objekts erzeugt wird. Ein Teilbereich 12 der aufzunehmenden Struktur 6, also des Unterkiefers, ist in der 3D-Röntgenaufnahme 9 gestrichelt dargestellt. Eine vorgegebene Aufnahmelagebeziehung 13 der Röntgenquelle 4 ist mit einer durchgezogenen Linie dargestellt. Gegenüber ist eine vorgegebene Aufnahmelagebeziehung 14 des Röntgendetektors 5 ebenfalls mit einer durchgezogenen Linie dargestellt. Dazu verschoben ist eine tatsächliche Aufnahmelagebeziehung 15 der Röntgenquelle 4 für den jeweiligen Aufnahmewinkel 16 gestrichelt dargestellt. Eine ebenfalls verschobene tatsächliche Aufnahmelagebeziehung 17 des Röntgendetektors 5 ist ebenfalls gestrichelt dargestellt. Die Röntgenstrahlen 18 in der Form eines kegelförmigen Strahlenkegels sind demnach ebenfalls verschoben und nehmen das Objekt 2 aus einem tatsächlichen Aufnahmewinkel 19 auf, der von dem vorgegebenen Aufnahmewinkel 16 deutlich abweicht. Diese Abweichung kann beispielsweise durch die Verstellung der Mechanik oder der Antriebe sowie durchzunehmende Reibkräfte in den Antrieben des Röntgengerätes 1 verursacht werden. Das vorliegende Verfahren dient also dazu, diese Verschiebung, die durch den Pfeil 20 angedeutet ist, zu kompensieren. Diese Verschiebung zwischen der vorgegebenen Aufnahmelagebeziehung 13, 14 und der tatsächlichen Aufnahmelagebeziehung 15, 17 kann auch in radialer Richtung bezüglich des Rotationspunktes 10 erfolgen. Die einzelnen Aufnahmelagebeziehungen 13 der Röntgenquelle 4 und die vorgegebenen Aufnahmelagebeziehungen 14 des Röntgendetektors 5 für alle Aufnahmewinkel 11 bilden dann eine Umlaufbahn 21, die mit einer durchgezogenen Linie dargestellt ist. Nach der Durchführung des Optimierungsverfahrens für jeden einzelnen Aufnahmewinkel 11 werden die tatsächlichen Aufnahmelagebeziehungen 15 der Röntgenquelle 4 und die tatsächlichen Aufnahmelagebeziehungen 17 des Röntgendetektors 5 ermittelt, die eine tatsächliche Umlaufbahn 22 bilden, die gestrichelt dargestellt ist. Die Abweichungen zwischen der vorgegebenen Umlaufbahn 21 und der tatsächlichen Umlaufbahn 22 können nicht nur durch die Störungen der Antriebsmechanik, sondern auch durch Bewegungen des Patienten während eines Umlaufs verursacht werden. Bei einer ursprünglichen Rekonstruktion unter Verwendung der vorgegebenen Umlaufbahn 21, die beispielsweise durch eine Werkskalibrierung vorgegeben sein kann, wird also eine unscharfe 3D-Röntgenaufnahme rekonstruiert, die insbesondere die aufzunehmende Struktur 6 unscharf darstellt, da darin Störungsartefakte enthalten sind. Bei der Rekonstruktion unter Verwendung der ermittelten tatsächlichen Umlaufbahn 22 wird eine 3D-Röntgenaufnahme 9 erzeugt, die insbesondere die Details und die aufzunehmende Struktur 6, wie den Unterkiefer, scharf und deutlich abbildet. Die Darstellung der 3D-Röntgenaufnahme 9 und des 3D-Modells 7 wird mittels einer Anzeigevorrichtung 23, wie eines Monitors, angezeigt. Die Bilddaten des Röntgendetektors 5 wie die einzelnen 2D-Röntgenaufnahmen werden per Kabel oder auch drahtlos an einen Computer 24 übermittelt. Mittels des Computers 24 werden auch das Rekonstruktionsverfahren und das Optimierungsverfahren durchgeführt. Der Computer 24 ist mit den Eingabemitteln, wie einer Tastatur 25 und einer Maus 26, verbunden, um es einem Benutzer zu ermöglichen mittels eines Cursors 27 zu navigieren.

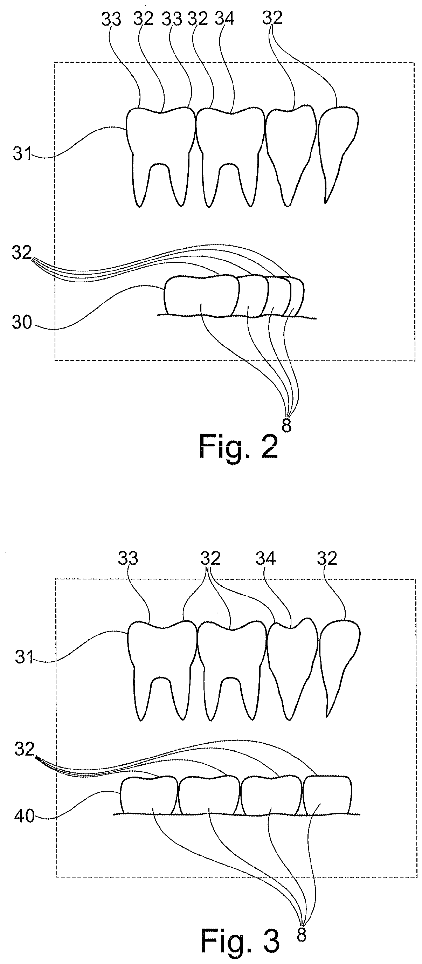

Die Fig. 2 zeigt eine Skizze zur Verdeutlichung des Optimierungsverfahrens. Unter Berücksichtigung der vorgegebenen Aufnahmelagebeziehung 13 der Röntgenquelle 4 und der vorgegebenen Aufnahmelagebeziehung 14 des Röntgendetektors 5 für den Aufnahmewinkel 16 wird durch die Anwendung eines Projektionsverfahrens auf das 3D-Modell 7 aus Fig. 1 eine virtuelle 2D-Projektionsaufnahme 30 erzeugt. Dabei wird also die Röntgenbestrahlung des virtuellen 3D-Modells 7 virtuell simuliert, indem ausgehend von einer virtuellen Röntgenquelle das 3D-Modell 7 durchstrahlt wird und nach einer simulierten Röntgenabsorption die 2D-Projektionsaufnahme 30 auf einem virtuellen Röntgendetektor abgebildet wird. Zum Vergleich ist die tatsächliche 2D-Röntgenaufnahme 31 aus dem tatsächlichen Aufnahmewinkel 19 dargestellt. Dabei ist lediglich der Teilbereich 12 mit den Backenzähnen 8 dargestellt. Es ist dabei deutlich sichtbar, dass die 2D-Projektionsaufnahme 30 des 3D-Modells 7 und damit der Backenzähne 8 in der Form deutlich von der dazugehörigen 2D-Röntgenaufnahme 31 abweicht. Dies wird durch die Verschiebung 20 zwischen dem vorgegebenen Aufnahmewinkel 16 und dem tatsächlichen Aufnahmewinkel 19 verursacht. Für das Vergleichsverfahren können insbesondere die Zahnränder 32 herangezogen werden, da diese sowohl in der 2D-Projektionsaufnahme 30 als auch in der 2D-Röntgenaufnahme 31 sichtbar sind. Für das Vergleichsverfahren können auch charakteristische Strukturen, wie Zahnhöcker 33 und Zahnvertiefungen 34, verwendet werden. Beim Vergleichsverfahren wird ein Ähnlichkeitsmaß bestimmt, dass ein zuverlässiges Maß für die Ähnlichkeit zweier Strukturen ist. Das Vergleichsverfahren kann mittels des Computers 24 aus Fig. 1 automatisch durchgeführt werden.

In Fig. 3 ist eine Skizze zur Verdeutlichung des Optimierungsverfahrens dargestellt, wobei im Vergleich zu Fig. 2 eine tatsächliche 2D-Projektionsaufnahme 40 unter Verwendung der tatsächlichen Lagebeziehung 15 der virtuellen Röntgenquelle und der tatsächlichen Aufnahmelagebeziehung 17 des virtuellen Röntgendetektors aus Fig. 1 durch die Verwendung des Projektionsverfahrens erzeugt wurde. Im Vergleich zu der 2D-Röntgenaufnahme 31 sieht man nun deutlich, dass die Zahnränder 32, die Zahnhöcker 33 sowie die Zahnvertiefungen 34 der Zähne 8 in ihrer Form übereinstimmen. Jede Abweichung der geometrischen Aufnahmelagebeziehung, auf deren Basis die virtuelle Projektionsaufnahme berechnet wurde, führt daher zu einem Ergebnis, das eine geringere Ähnlichkeit der Strukturen in der virtuellen Produktionsaufnahme und in der 2D-Röntgenaufnahme aufweist. Das Ähnlichkeitsmaß wird also maximal, sodass das Optimierungsverfahren beendet werden kann und damit die tatsächliche Aufnahmelagebeziehung der Röntgenquelle und des Röntgendetektors ermittelt wurde. Der Verlauf des Optimierungsverfahrens zwischen einer Startlösung in Fig. 2 und einer finalen Lösung in Fig. 3 läuft dabei schrittweise ab. Die Änderung 20 zwischen der vorgegebenen Aufnahmelagebeziehung 13 und der tatsächlichen Aufnahmelagebeziehung 15 kann beispielsweise mittels einer Transformationsmatrix beschrieben werden.

### Bezugszeichen

- 1: Röntgengerät
- 2: Aufzunehmendes Objekt
- 3: Röntgenstrahlen
- 4: Röntgenquelle
- 5: Röntgendetektor
- 6: Aufzunehmende Struktur
- 6: Unterkiefer
- 7: 3D-Modell
- 8: Backenzähne
- 9: 3D-Röntgenaufnahme
- 10: Rotationspunkt
- 11: Aufnahmewinkel
- 12: Teilbereich
- 13: Vorgegebene Aufnahmelagebeziehung
- 14: Vorgegebene Aufnahmelagebeziehung
- 15: Tatsächliche Aufnahmelagebeziehung
- 16: Aufnahmewinkel
- 17: Tatsächliche Aufnahmelagebeziehung
- 18: Röntgenstrahlen
- 19: Tatsächlicher Aufnahmewinkel
- 20: Pfeil
- 20: Verschiebung
- 21: Vorgegebene Umlaufbahn
- 22: Tatsächliche Umlaufbahn
- 23: Anzeigevorrichtung
- 24: Computer
- 25: Tastatur
- 26: Maus
- 27: Cursor
- 30: virtuelle 2D-Projektionsaufnahme
- 31: tatsächliche 2D-Röntgenaufnahme
- 32: Zahnränder
- 33: Zahnhöcker
- 34: Zahnvertiefungen
- 40: ermittelte virtuelle 2D-Projektionsaufnahme

## Patentansprüche

1. Verfahren zur Kalibrierung mindestens einer 2D-Röntgenaufnahme (31) eines aufzunehmenden Objekts (2), die mittels eines Röntgengeräts (1) aufgenommen wurde, indem Röntgenstrahlen (3), die mittels einer Röntgenquelle (4) erzeugt werden, das Objekt (2) durchstrahlen und mittels eines Röntgendetektors (5) aufgenommen werden, wobei ein bereits vorhandenes 3D-Modell (7) umfassend eine Struktur (6) des Objekts (2) mit der 2D-Röntgenaufnahme (31) verglichen wird,
wobei beim Vergleich des 3D-Modells (7) mit der 2D-Röntgenaufnahme (31) durch die Anwendung eines virtuellen Projektionsverfahrens auf mindestens einen Teil des 3D-Modells (7) unter Berücksichtigung einer vorgegebenen Aufnahmelagebeziehung (13, 14) eine virtuelle 2D-Projektionsaufnahme (30, 40) erzeugt wird, wobei die Struktur (6) in der 2D-Röntgenaufnahme (31) mit der Struktur (6) in der virtuellen 2D-Projektionsaufnahme (30, 40) verglichen wird, wobei beim Vergleich der Struktur (6) in der 2D-Röntgenaufnahme (31) mit der virtuellen 2D-Projektionsaufnahme (30, 40) Abweichungen festgestellt werden, **dadurch gekennzeichnet, dass** die vorgegebene Aufnahmelagebeziehung (13, 14) Schritt für Schritt im Rahmen eines Optimierungsverfahrens geändert wird und nach jeder Änderung eine erneute virtuelle 2D-Projektionsaufnahme (40) erzeugt wird, bis die Abweichungen kleiner als ein festgelegter Schwellenwert sind, so dass die ermittelte Aufnahmelagebeziehung der tatsächlichen Aufnahmelagebeziehung (15, 17) der Röntgenquelle (4) und des Röntgendetektors (5) relativ zum Objekt (2) und/oder relativ zueinander entspricht.

2. Verfahren nach Anspruch 1, **dadurch gekennzeichnet, dass** die 2D-Röntgenaufnahmen (31) bei einer Bewegung der Röntgenquelle (4) und des Röntgendetektors (5) um das Objekt (2) schrittweise aus unterschiedlichen Aufnahmewinkeln (11) aufgenommen werden, wobei aus den aufgenommenen 2D-Röntgenaufnahmen (31) durch die Anwendung eines Rekonstruktionsverfahrens in Kenntnis der ermittelten Aufnahmelagebeziehungen (15, 17) der 2D-Röntgenaufnahmen (31) eine 3D-Röntgenaufnahme (9) des Objekts (2) oder eine Panoramaschichtaufnahme des Objekts (2) erzeugt wird.

3. Verfahren nach Anspruch 1 oder 2, **dadurch gekennzeichnet, dass** die Struktur (6) des Objekts (2) ein Oberkiefer, ein Unterkiefer (6), eine Gruppe von Zähnen, ein Zahnersatzteil, eine Füllung, ein Inlay, das gesamte Objekt, einen Teil des Objekts, ein Patientenkopf und/oder ein einzelner Zahn ist.

4. Verfahren nach einem der Ansprüche 1 bis 3, **dadurch gekennzeichnet, dass** das vorhandene 3D-Modell (7) der Struktur (6, 8) mittels eines optischen dreidimensionalen Oberflächenvermessungsverfahrens aufgenommen ist, wobei das 3D-Modell (7) lediglich eine Oberfläche der Struktur (6, 8) enthält, wobei beim Vergleich ein Oberflächenrand (33) der Struktur (6, 8) in der 2D-Röntgenaufnahme (31) mit einem Oberflächenrand (33) der Struktur (6, 8) in der virtuellen 2D-Projektionsaufnahme (30, 40) verglichen wird.

5. Verfahren nach einem der Ansprüche 1 bis 3, **dadurch gekennzeichnet, dass** das vorhandene 3D-Modell (7) der Struktur (6) erzeugt wurde, indem ein Abdruck der Struktur (6, 8) mittels eines optischen dreidimensionalen Oberflächenvermessungsverfahrens aufgenommen wurde, wobei das 3D-Modell (7) lediglich eine Oberfläche der Struktur enthält, wobei beim Vergleich ein Oberflächenrand der Struktur in der 2D-Röntgenaufnahme (31) mit einem Oberflächenrand der Struktur in der virtuellen 2D-Projektionsaufnahme (30) verglichen wird.

6. Verfahren nach einem der Ansprüche 1 bis 3, **dadurch gekennzeichnet, dass** das vorhandene 3D-Modell (7) der Struktur (6) mittels eines dreidimensionalen Volumenvermessungsverfahrens, insbesondere mittels eines MRT-Verfahrens, CT-Verfahrens oder DVT-Verfahrens, aufgenommen wurde und Volumendaten der Struktur enthält, wobei beim Vergleich die Struktur in der 2D-Röntgenaufnahme (31) mit der simulierten Projektion der Struktur in der virtuellen 2D-Projektionsaufnahme (30)verglichen wird.

7. Verfahren nach einem der Ansprüche 1 bis 6, **dadurch gekennzeichnet, dass** beim virtuellen Projektionsverfahren nicht nur die Aufnahmelagebeziehung (13, 14, 15, 17) der Röntgenquelle (4) und des Röntgendetektors (5) relativ zur Objekt (2), sondern auch die Dicke der aufzunehmenden Struktur (6, 8) und damit die Röntgenschwächung durch die Struktur (6, 8) und/oder das Material der Struktur (6, 8) und damit die davon abhängige Röntgenschwächung berücksichtigt werden.

8. Verfahren nach einem der Ansprüche 1 bis 7, **dadurch gekennzeichnet, dass** bei der Durchführung des Optimierungsverfahrens als Startlösung die vorgegebene Aufnahmelagebeziehung (13, 14) aus einer bekannten Kalibrierung verwendet wird.

9. Verfahren nach einem der Ansprüche 1 bis 8, **dadurch gekennzeichnet, dass** beim Vergleich der 2D-Röntgenaufnahme (31) mit der virtuellen 2D-Projektionsaufnahme (30, 40) zur Bestimmung der Abweichungen ein Ähnlichkeitsmaß berechnet wird, wobei ein Gradienten-Differenzen-Verfahren, ein direktes Differenzen-Verfahren, ein Korrelationen-Verfahren, ein Kreuzkorrelationen-Verfahren erster und/oder höherer Ordnung, ein statistisches Verfahren oder ein Verfahren der geringsten quadratischen Fehler verwendet wird.

10. Verfahren nach einem der Ansprüche 1 bis 9, **dadurch gekennzeichnet, dass** in einem ersten Schritt eine erste Struktur (6, 8) des Objekts (2) gewählt wird, um nach dem vorliegenden Verfahren die ersten tatsächlichen Aufnahmelagebeziehungen der 2D-Röntgenaufnahmen (31) für diese erste Struktur zu bestimmen, wobei in einem zweiten Schritt eine zweite Struktur des Objekts gewählt wird, um nach dem vorliegenden Verfahren die zweiten tatsächlichen Aufnahmelagebeziehungen der 2D-Röntgenaufnahmen (31) für diese zweite Struktur zu bestimmen.

11. Verfahren nach Anspruch 10, **dadurch gekennzeichnet, dass** unter Verwendung der ersten tatsächlichen Aufnahmelagebeziehungen eine erste 3D-Röntgenaufnahme (9) rekonstruiert wird und unter Verwendung der zweiten tatsächlichen Aufnahmelagebeziehungen eine zweite 3D-Röntgenaufnahme rekonstruiert wird, wobei anschließend ein erster Bereich in der ersten 3D-Röntgenaufnahme, der die erste Struktur (6, 8) scharf abbildet, mit einem zweiten Bereich in der zweiten 3D-Röntgenaufnahme, der die zweite Struktur scharf abbildet, zu einer gesamten 3D-Röntgenaufnahme des Objekts (2) zusammengefügt werden.

12. Verfahren nach Anspruch 10 oder 11, **dadurch gekennzeichnet, dass** die erste Struktur ein Unterkiefer oder ein Teil des Unterkiefers und die zweite Struktur ein Oberkiefer oder ein Teil des Oberkiefers sind.

## Claims

1. Method for calibrating at least one 2D X-ray image (31) of an object (2) to be recorded, which is recorded by means of an X-ray device (1) in that X-rays (3) produced by an X-ray source (4) radiate through the object (2) and are recorded by means of an X-ray detector (5), wherein an already existing 3D model (7) comprising a structure (6) of the object (2) is compared to the 2D X-ray image (31), wherein,
for the comparison of the 3D model (7) with the 2D X-ray image (31) and taking into account a predetermined image positional relationship (13, 14), a virtual 2D projection image (30, 40) is produced by applying a virtual projection method to at least one part of the 3D model (7), wherein the structure (6) in the 2D X-ray image (31) is compared to the structure (6) in the virtual 2D projection image (30, 40), wherein deviations are identified during the comparison of the structure (6) in the 2D X-ray image (31) with the virtual 2D projection image (30, 40), **characterized in that**
the image positional relationship (13, 14) is incrementally changed within the framework of an optimization method and a new virtual 2D projection image (40) is generated after each change until the deviations are smaller than a defined threshold value, so that the determined image positional relationship corresponds to the actual image positional relationship (15, 17) of the X-ray source (4) and the X-ray detector (5) relative to the object (2) and/or relative to each other.

2. Method according to Claim 1, **characterized in that** the 2D X-ray images (31) are recorded step-by-step from different recording angles (11) during a movement of the X-ray source (4) and the X-ray detector (5) around the object (2), wherein, by using a reconstruction method and knowing the determined image positional relationships (15, 17) of the 2D X-ray images (31), a 3D X-ray image (9) of the object (2), or a panoramic tomographic image of the object (2), is generated from the recorded 2D X-ray images (31).

3. Method according to Claim 1 or 2, **characterized in that** the structure (6) of the object (2) is an upper jaw, a lower jaw (6), a group of teeth, a tooth replacement part, a filling, an inlay, the entire object, a part of the object, the head of a patient and/or a single tooth.

4. Method according to any one of Claims 1 to 3, **characterized in that** the existing 3D model (7) of the structure (6, 8) is recorded by means of an optical three-dimensional surface measurement method, wherein the 3D model (7) includes only one surface of the structure (6, 8), wherein, during the comparison, a surface edge (33) of the structure (6, 8) in the 2D X-ray image (31) is compared with a surface edge (33) of the structure (6, 8) in the virtual 2D projection image (30, 40).

5. Method according to any one of Claims 1 to 3, **characterized in that** the existing 3D model (7) of the structure (6) was generated by recording an impression of the structure (6, 8) with the aid of an optical three-dimensional surface measurement method, wherein the 3D model (7) includes only one surface of the structure, wherein, during the comparison, a surface edge of the structure in the 2D X-ray image (31) is compared with a surface edge of the structure in the virtual 2D projection image (30).

6. Method according to any one of Claims 1 to 3, **characterized in that** the existing 3D model (7) of the structure (6) has been recorded by means of a three-dimensional volume measurement method, in particular by means of an MRI method, CT method or DVT method, and includes volume data of the structure, wherein, for the comparison, the structure in the 2D X-ray image (31) is compared with the simulated projection of the structure in the virtual 2D projection image (30).

7. Method according to any one of Claims 1 to 6, **characterized in that** not only the image positional relationship (13, 14, 15, 17) of the X-ray source (4) and the X-ray detector (5) relative to the object (2) is taken into account for the virtual projection method, but also the thickness of the structure (6, 8) to be recorded, thus also taking into account the X-ray attenuation caused by the structure (6, 8) and/or the material of the structure (6, 8) and the X-ray attenuation dependent thereon.

8. Method according to any one of Claims 1 to 7, **characterized in that** the predetermined image positional relationship (13, 14) from a known calibration is used as the starting solution for the implementation of the optimization method.

9. Method according to any one of Claims 1 to 8, **characterized in that**, when comparing the 2D X-ray image (31) with the virtual 2D projection image (30, 40), a degree of similarity is calculated for the determination of the deviations, wherein a gradient difference method, a direct difference method, a correlation method, a cross-correlation method of the first and/or a higher order, a statistical method or a least squares errors method is used.

10. Method according to any one of Claims 1 to 9, **characterized in that**, in a first step, a first structure (6, 8) of the object (2) is selected to determine the first actual image positional relationships of the 2D X-ray images (31) for this first structure according to the present method, wherein, in a second step, a second structure of the object is selected to determine the second actual image positional relationships of the 2D X-ray images (31) for this second structure according to the present method.

11. Method according to Claim 10, **characterized in that** a first 3D X-ray image (9) is reconstructed using the first actual image positional relationships and a second 3D X-ray image is reconstructed using the second actual image positional relationships, wherein a first region in the first 3D X-ray image, which displays the first structure (6, 8) in sharp focus, is subsequently merged with a second region in the second 3D X-ray image, which displays the second structure in sharp focus, to form one overall 3D X-ray image of the object (2).

12. Method according to Claim 10 or 11, **characterized in that** the first structure is a lower jaw or a part of the lower jaw and the second structure is an upper jaw or a part of the upper jaw.

## Revendications

1. Procédé pour la calibration d'au moins une radiographie 2D (31) d'un objet à radiographier (2), laquelle a été prise au moyen d'un appareil radiographique (1), des rayons X (3) générés au moyen d'une source de rayons X (4) traversant l'objet (2) et étant enregistrés au moyen d'un détecteur de rayons X (5), un modèle 3D (7) déjà existant comprenant une structure (6) de l'objet (2) étant comparé à la radiographie 2D (31), une vue de projection 2D virtuelle (30, 40) étant générée lors de la comparaison du modèle 3D (7) à la radiographie 2D (31) par l'utilisation d'un procédé de projection virtuelle sur au moins une partie du modèle 3D (7) tout en tenant compte d'une relation de position d'enregistrement (13, 14) prédéfinie, la structure (6) dans la radiographie 2D (31) étant comparée à la structure (6) dans la vue de projection 2D virtuelle (30, 40), des différences étant détectées lors de la comparaison de la structure (6) dans la radiographie 2D (31) à la vue de projection 2D virtuelle (30, 40), **caractérisé en ce que** la relation de position d'enregistrement (13, 14) prédéfinie
est modifiée pas à pas dans le cadre d'un procédé d'optimisation et qu'après chaque modification une nouvelle vue de projection 2D virtuelle (40) est générée, jusqu'à ce que les différences soient inférieures à une valeur de seuil définie, de telle sorte que la relation de position d'enregistrement déterminée correspond à la relation de position d'enregistrement réelle (15, 17) de la source de rayons X (4) et du détecteur de rayons X (5) par rapport à l'objet (2) et/ou entre eux.

2. Procédé selon la revendication 1, **caractérisé en ce que** les radiographies 2D (31) sont prises par étape à partir de différents angles d'enregistrement (11) lors d'un mouvement de la source de rayons X (4) et du détecteur de rayons X (5) autour de l'objet (2), une radiographie 3D (9) de l'objet (2) ou une radiographie panoramique de l'objet (2) étant générée à partir des radiographies 2D (31) enregistrées par l'utilisation d'un procédé de reconstruction en connaissance des relations de position d'enregistrement (15, 17) des radiographies 2D (31).

3. Procédé selon la revendication 1 ou 2, **caractérisé en ce que** la structure (6) de l'objet (2) est un maxillaire supérieur, un maxillaire inférieur (6), un groupe de dents, une prothèse dentaire, un plombage, un inlay, l'objet entier, une partie de l'objet, une tête de patient et/ou une dent individuelle.

4. Procédé selon l'une quelconque des revendications 1 à 3, **caractérisé en ce que** le modèle 3D (7) existant de la structure (6, 8) a été enregistré au moyen d'un procédé de mesure de surface tridimensionnel optique, le modèle 3D (7) contenant uniquement une surface de la structure (6, 8), un pourtour de surface (33) de la structure (6, 8) dans la radiographie 2D (31) étant comparé à un pourtour de surface (33) de la structure (6, 8) dans la vue de projection 2D virtuelle (30, 40) lors de la comparaison.

5. Procédé selon l'une quelconque des revendications 1 à 3, **caractérisé en ce que** le modèle 3D (7) existant de la structure (6) a été généré par l'enregistrement d'un moulage de la structure (6, 8) au moyen d'un procédé de mesure de surface tridimensionnel optique, le modèle 3D (7) contenant uniquement une surface de la structure, un pourtour de surface de la structure dans la radiographie 2D (31) étant comparé à un pourtour de surface de la structure dans la vue de projection 2D virtuelle (30) lors de la comparaison.

6. Procédé selon l'une quelconque des revendications 1 à 3, **caractérisé en ce que** le modèle 3D (7) existant de la structure (6) a été enregistré au moyen d'un procédé de mesure de volume tridimensionnel, en particulier au moyen d'un procédé d'IRM, d'un procédé de CT ou d'un procédé de DVT, et qu'il contient des données de volume de la structure, la structure dans la radiographie 2D (31) étant comparée à la projection simulée de la structure dans la vue de projection 2D virtuelle (30) lors de la comparaison.

7. Procédé selon l'une quelconque des revendications 1 à 6, **caractérisé en ce que** dans le procédé de projection virtuelle non seulement la relation de position d'enregistrement (13, 14, 15, 17) de la source de rayons X (4) et du détecteur de rayons X (5) par rapport à l'objet (2) mais aussi l'épaisseur de la structure (6, 8) à enregistrer sont prises en compte, et donc l'affaiblissement des rayons X par la structure (6, 8) et/ou par le matériau de la structure (6, 8) et donc l'affaiblissement des rayons X qui en dépend.

8. Procédé selon l'une quelconque des revendications 1 à 7, **caractérisé en ce que** la relation de position d'enregistrement (13, 14) prédéfinie provenant d'un calibrage connu est utilisée comme solution de départ lors de l'exécution du procédé d'optimisation.

9. Procédé selon l'une quelconque des revendications 1 à 8, **caractérisé en ce qu'**une mesure de similitude est calculée lors de la comparaison de la radiographie 2D (31) à la vue de projection 2D virtuelle (30, 40), un procédé de différences de gradients, un procédé de différences direct, un procédé de corrélation, un procédé de corrélation croisée de premier ordre et/ou d'ordre supérieur, un procédé statistique ou un procédé d'erreur quadratique minimale étant utilisé.

10. Procédé selon l'une quelconque des revendications 1 à 9, **caractérisé en ce qu'**une première structure (6, 8) de l'objet (2) est sélectionnée dans une première étape, pour déterminer selon le présent procédé les premières relations de position d'enregistrement réelles des radiographies 2D (31) pour cette première structure, une deuxième structure de l'objet étant sélectionnée dans une deuxième étape pour déterminer selon le présent procédé les deuxièmes relations de position d'enregistrement réelles des radiographies 2D (31) pour cette deuxième structure.

11. Procédé selon la revendication 10, **caractérisé en ce qu'**une première radiographie 3D (9) est reconstruite en utilisant les premières relations de position d'enregistrement réelles, et une deuxième radiographie 3D est reconstruite en utilisant les deuxièmes relations de position d'enregistrement réelles, une première zone dans la première radiographie 3D, qui reproduit avec netteté la première structure (6, 8) étant ensuite assemblée avec une deuxième zone dans la deuxième radiographie 3D, qui reproduit avec netteté la deuxième structure, en une radiographie 3D globale de l'objet (2) .

12. Procédé selon la revendication 10 ou 11, **caractérisé en ce que** la première structure est un maxillaire inférieur ou une partie du maxillaire inférieur et que la deuxième structure est un maxillaire supérieur ou une partie du maxillaire supérieur.
